(19) Europäisches Patentamt
European Patent Office
Office européen des brevets

(11) **EP 4 397 261 B1**

(12) **EUROPEAN PATENT SPECIFICATION**

(45) Date of publication and mention
of the grant of the patent:
**24.12.2025 Bulletin 2025/52**

(51) International Patent Classification (IPC):
***A61B 18/12*** (2006.01)     ***A61B 18/14*** (2006.01)
***A61B 18/00*** (2006.01)

(21) Application number: **22863466.3**

(22) Date of filing: **30.08.2022**

(52) Cooperative Patent Classification (CPC):
**A61B 18/14; A61B 18/1206; A61B 18/1492;**
A61B 2018/00577; A61B 2018/00642;
A61B 2018/00666; A61B 2018/00672;
A61B 2018/00678; A61B 2018/00702;
A61B 2018/00708; A61B 2018/00767;
A61B 2018/00779; A61B 2018/00827;
A61B 2018/00875; A61B 2018/00892;     (Cont.)

(86) International application number:
**PCT/CN2022/115891**

(87) International publication number:
**WO 2023/030331 (09.03.2023 Gazette 2023/10)**

(54) **PULSE MONITORING DEVICE AND STORAGE MEDIUM**

PULSÜBERWACHUNGSVORRICHTUNG UND SPEICHERMEDIUM

DISPOSITIF DE SURVEILLANCE D'IMPULSIONS ET SUPPORT DE STOCKAGE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.09.2021 CN 202111020857**

(43) Date of publication of application:
**10.07.2024 Bulletin 2024/28**

(73) Proprietor: **Hangzhou Wknife Medical Technology
Co., Ltd.
Hangzhou, Zhejiang 310018 (CN)**

(72) Inventors:
• **ZHONG, Xinghua
Hangzhou, Zhejiang 310018 (CN)**
• **WANG, Long
Hangzhou, Zhejiang 310018 (CN)**
• **YANG, Ke
Hangzhou, Zhejiang 310018 (CN)**

(74) Representative: **Monteiro Alves, Inês
Alameda Dos Oceanos, № 41K-21
Parque das Nações
1990-207 Lisboa (PT)**

(56) References cited:
EP-A1- 1 769 762          CN-A- 109 124 760
CN-A- 109 157 280          CN-A- 109 157 280
CN-A- 112 540 221          CN-A- 112 540 221
CN-A- 112 842 516          CN-A- 113 648 053
US-A1- 2016 066 977          US-A1- 2020 289 185

(52) Cooperative Patent Classification (CPC): (Cont.)
A61B 2018/00898

# EP 4 397 261 B1

**Description**

## TECHNICAL FIELD

The present application relates to the field of medical device technologies, in particular to a pulse monitoring device, and a computer-readable storage medium.

## BACKGROUND

Pulse ablation is an emerging biological tissue ablation technology that is applicable to the treatment of clinical diseases such as tumor ablation, cardiac tissue ablation, and hyperplasia tissue ablation.

Currently, during a pulse ablation process, real-time monitoring and evaluation of ablation effects of a target biological tissue have not been considered. Consequently, it is unable to guide the pulse ablation process based on the state changes of the target biological tissue. CN 109157280 A discloses dynamic real-time evaluation equipment for irreversible electroporation tissue ablation, which utilizes an electrode in an irreversible electroporation tumor treatment primary pulse application process to detect tissue impedance spectrum changes before and after pulse treatment.

## SUMMARY

In view of shortcomings in the prior art, the present application provides a pulse monitoring device, and a computer-readable storage medium, as defined in the appended set of claims, so as to solve the technical problem in the prior art that it is unable to guide the process of pulse ablation according to changes in biological tissue. The invention is defined by the claims. In the following any method of monitoring a treatment and/or including treatment steps, and in particular ablation steps, is not claimed and is disclosed for illustrative purposes only.

The technical solutions in the embodiments of the present application at least include the following beneficial technical effects.

In the pulse monitoring method of the embodiments of the present application, it is able to apply a first pulse sequence and a second pulse sequence to target biological tissue, and determine whether a termination condition for the first pulse sequence is met according to a feedback signal after the second pulse sequence is applied to the target biological tissue. That is, during a pulse ablation process, it is able to determine an ablation status of the target biological tissue based on the feedback signal, so as to monitor changes in the target biological tissue during the pulse ablation process in real time. When it is determined that a termination condition for the first pulse sequence is met, the output of the first pulse sequence is stopped. That is, in the embodiments of the present application, it is able to guide the pulse ablation process according to the state change of the biological tissue. After determining that the pulse ablation effect is achieved, the output of the first pulse sequence for ablating the biological tissue is stopped, thereby to guide the pulse ablation process according to the ablation status of the target biological tissue.

The additional aspects and advantages of the present disclosure will be given or may become apparent in the following description, or may be understood through the implementation of the present application.

## BRIEF DESCRIPTION OF THE DRAWINGS

The above and/or additional aspects as well as advantages of the present application will become apparent and are easily understood in the following description with reference to the following drawings. In these drawings,

Fig. 1 is a schematic structural diagram of a pulse monitoring device according to the embodiments of the present application;

Fig. 2 is another schematic structural diagram of the pulse monitoring device according to the embodiments of the present application;

Fig. 3 is a schematic diagram of a circuit structure in the pulse monitoring device according to the embodiments of the present application;

Fig. 4 is a schematic flow chart of a pulse monitoring method according to the illustrative embodiments of the present application not in accordance with the appended claims;

Fig. 5 is a waveform diagram of an outputted pulse sequence according to the embodiments of the present application; and

Fig. 6 is a schematic diagram of a pulse monitoring apparatus according to the illustrative embodiments of the present application not in accordance with the appended claims.

Reference Sign List

10-pulse monitoring device
100-pulse generating circuit, 110-first pulse generating circuit, 111-first pulse generating unit, 120-second pulse generating circuit, 121-second pulse generating unit. 200-control unit
300-monitoring unit
112-first capacitor discharge relay, 122-second capacitor discharge relay;
310-first Pearson coil, 320-second Pearson coil, 150-first output relay, 160-second output relay, 170-foot switch.
RLoad-load
UH-first power source, UL-second power source.
400-display unit
500-alarm unit.

**DETAILED DESCRIPTION**

The present application will be described hereinafter in conjunction with the embodiments and the drawings. Identical or similar reference numbers in the drawings represent an identical or similar element or elements having an identical or similar function. In addition, the detailed description about any known technology will be omitted when it is unnecessary to the features in the present application. The following embodiments are for illustrative purposes only, but shall not be used to limit the scope of the present application as defined by the appended claims.

As can be appreciated by a person skilled in the art, unless otherwise defined, all terms (including technical and scientific terms) used herein have the same meaning as commonly understood by one of ordinary skill in the art to which the present application is a part. Terms, such as those defined in commonly used dictionaries, should be interpreted as having a meaning that is consistent with their meaning in the context of the relevant art and will not be interpreted in an idealized or overly formal sense, unless expressly so defined herein.

As used herein, the singular forms, "a," "an," and "the" are intended to include the plural forms as well, unless the context clearly indicates otherwise. Moreover, the terms "comprises," "comprising," "includes," and/or "including," when used in this specification, specify the presence of stated features, integers, steps, operations, elements, components, and/or groups thereof, but do not preclude the presence or addition of one or more other features, integers, steps, operations, elements, components, and/or groups thereof. In the case that one element is connected or coupled to another element, it may be directly connected or coupled to the other element, or an intermediate element may be arranged therebetween. At this time, the element may be connected or coupled to the other element in a wireless or wired manner. In addition, the expression "and/or" is used to indicate the existence of all or any one of one or more of listed items, or combinations thereof.

Through research, it has been found that, in a conventional pulse ablation process, real-time monitoring and evaluation of the pulse ablation effects have not been considered. Consequently, it is unable to obtain the changes in biological tissue during the ablation process, and thus there is no basis for precisely guiding the pulse ablation progress.

It has been further found through research that most of the conventional pulse ablation technologies only focus on lesion location, and there is no disclosed method or technology that can monitor the pulse ablation process in real time. Moreover, in order to realize a monitoring function for the existing pulse generating apparatus, other monitoring devices or independent modules need to be equipped. Therefore, further exploration is needed to achieve monitoring of pulse ablation.

The present application provides a pulse monitoring method, apparatus and device, and a storage medium, so as to solve the above technical problems in the prior art.

The technical solutions of the present application and how the technical solutions of the present application solve the above technical problems will be described in detail below with reference to specific embodiments.

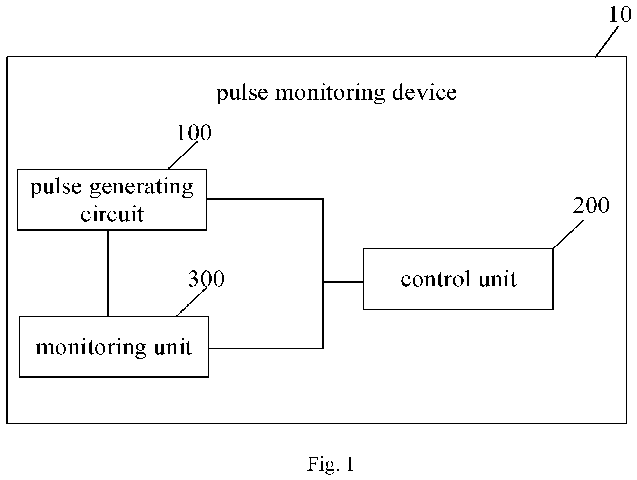

The embodiments of the present application provide a pulse monitoring device 10. As shown in Fig. 1, the pulse monitoring device 10 includes: a pulse generating circuit 100, a control unit 200 and a monitoring unit 300.

The control unit 200 is communicatively connected to the pulse generating circuit 100, and configured to control the pulse generating circuit 100 to apply a first pulse sequence and a second pulse sequence to target biological tissue. A voltage of the second pulse sequence is smaller than a voltage of the first pulse sequence, and the first pulse sequence is used to ablate the target biological tissue. The control unit is further configured to determine, according to a feedback signal forwarded by the monitoring unit 300, whether a termination condition for the first pulse sequence is met, and output a termination instruction for the first pulse sequence to the pulse generating circuit 100 when it is determined that the termination condition for the first pulse sequence is met.

The monitoring unit 300 is communicatively connected to the control unit 200, and configured to obtain the feedback signal after the second pulse sequence is applied to the target biological tissue, and output the feedback signal to the control unit 200.

The pulse generation circuit 100 of the pulse monitoring device 10 in the embodiments of the present application may apply the first pulse sequence and the second pulse sequence to the target biological tissue under the control of the control unit 200, and the control unit 200 determines according to the feedback signal from the monitoring unit 300 whether the termination condition for the first pulse sequence is met. That is, during a pulse ablation process, the control unit 200 may determine an ablation status of the target biological tissue based on the feedback signal, so as to monitor the ablation status of the target biological tissue during the pulse ablation process in real time.

The control unit 200 in the embodiments of the present application can guide the pulse ablation process according to the state change of the biological tissue. After determining that the pulse ablation effect is achieved, the control unit 200 outputs a termination instruction for the first pulse sequence, so that the control unit 200 controls the pulse generation circuit 100 to stop outputting the first pulse sequence for biological tissue ablation, thereby to guide the pulse ablation process according to changes in biological tissue.

Through the pulse monitoring device 10 in the embodiments of the present application, it realizes that two functions of pulse ablation and monitoring are integrated into one device, and thereby it does not need to be equipped with other monitoring devices or independent modules, making it convenient to use and operate.

Optionally, the target biological tissue includes a portion of a human body to be ablated.

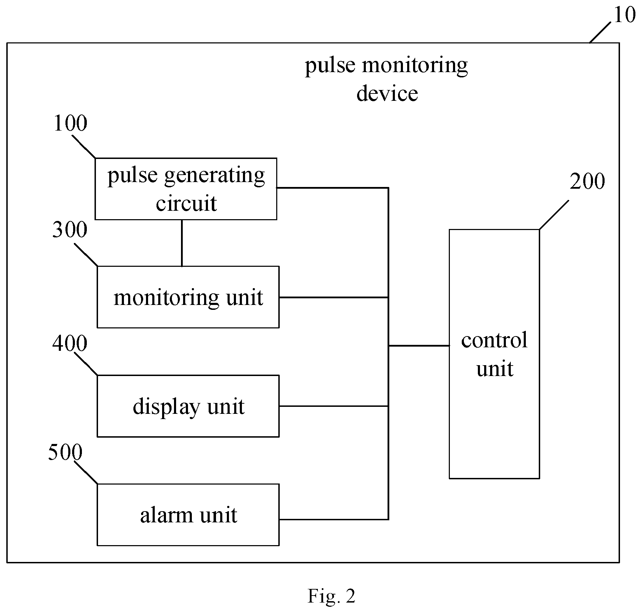

Optionally, as shown in Fig. 2, the pulse monitoring device 10 further includes a display unit 400 communicatively connected to the control unit 200 and configured to display a real-time impedance value.

Optionally, the display unit 400 is further configured to display a real-time impedance value curve, where the real-time impedance value curve comprises at least two real-time impedance values in a set time period, or, display the real-time impedance value and corresponding biological indicator information of the target biological tissue, where the biological indicator information comprises at least one of the following: heart rate, blood pressure or blood oxygen concentration.

Optionally, the display unit 400 may be a display screen, used to display such information as the real-time impedance value, the real-time impedance value curve, or biological indicator information.

Optionally, as shown in Fig. 2, the pulse monitoring device 10 further includes an alarm unit 500. The alarm unit 500 is communicatively connected to the control unit 200 and configured to issue an alarm prompt when the biological indicator information of the target biological tissue is greater than a set threshold. The alarm prompt includes emitting an alarm sound and/or outputting alarm information to the control unit 200. The control unit 200 is configured to output a termination instruction for the first pulse sequence in response to receiving the alarm information.

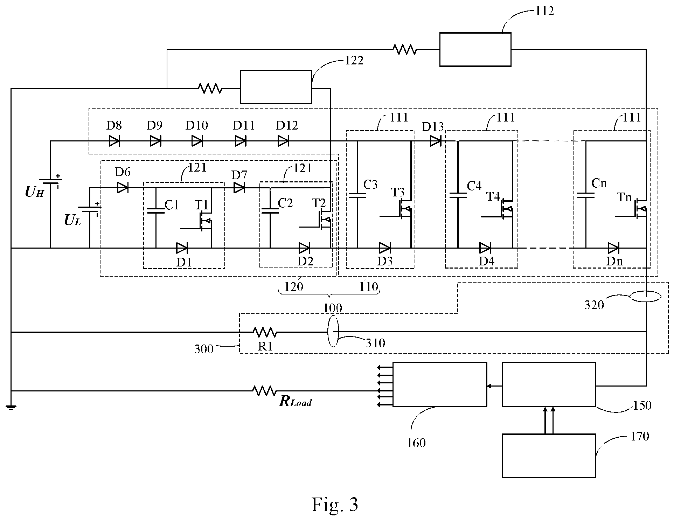

As shown in Fig. 3, the pulse generating circuit 100 includes a first pulse generating circuit 110 for outputting the first pulse sequence and a second pulse generating circuit 120 for outputting the second pulse sequence. The first pulse generating circuit 110 and the second pulse generating circuit 120 are integrated on a same circuit board.

In the present application, the first pulse generating circuit 110 and the second pulse generating circuit 120 are integrated on the same circuit board, so it allows a single circuit board to output the first pulse sequence and the second pulse sequence, accommodating both ablation and monitoring functionalities.

As shown in Fig. 3, the first pulse generating circuit 110 includes at least one level of first pulse generating units 111 electrically connected sequentially.

Each first pulse generating unit 111 is electrically connected to the control unit 200, and configured to be turned on under control of the control unit 200 and apply the first pulse sequence to the target biological tissue.

As shown in Fig. 3, each of the at least one level of first pulse generating units 111 includes a first capacitor, a first switching device and a first diode, a first terminal of the first capacitor is electrically connected to a first terminal of the first switching device, a positive electrode and a negative electrode of the first diode are electrically connected to a second terminal of the first capacitor and a second terminal of the first switching device respectively, and a control terminal of the first switching device is electrically connected to the control unit 200.

Optionally, when the first pulse generating circuit 110 discharges, the control unit 200 controls the first switching device of the first pulse generating unit 111 to be turned on.

Optionally, as shown in Fig. 3, the first pulse generating circuit 110 further includes at least one second diode; a first terminal of a first capacitor in a first level of first pulse generating unit 110 is electrically connected to a first power source UH through the at least one second diode. An anode of the second diode is electrically connected to the first power source UH, and a cathode of the second diode is electrically connected to the first terminal of the first capacitor in the first level of first pulse generating unit 110.

Optionally, as shown in Fig. 3, the first pulse generating circuit 110 further includes at least one third diode, an anode and a cathode of the third diode are electrically connected to two adjacent first pulse generating units 111 respectively. The anode and the cathode of the third diode are electrically connected to a first terminal of a first switching device in a previous level of first pulse generating unit and a first terminal of a first capacitor in a next level of first pulse generating unit.

In some embodiments, as shown in Fig. 3, the second pulse generating circuit 120 includes at least one level of second pulse generating units 121 electrically connected sequentially.

Each second pulse generating unit 121 is electrically connected to the control unit 200 and configured to be turned on under control of the control unit 200 and apply the second pulse sequence to the target biological tissue.

Optionally, as shown in Fig. 3, the second pulse generating unit 121 includes a second capacitor, a second switching device and a fourth diode. A first terminal of the second capacitor is electrically connected to a first terminal of the second switching device, a positive electrode and a negative electrode of the fourth diode are electrically connected to a second terminal of the second capacitor and a second terminal of the second switching device respectively, and a control terminal of the second switching device is electrically connected to the control unit 200.

Optionally, when the second pulse generating circuit 120 discharges, the control unit 200 controls the second switching device of the second pulse generating unit 121 to be turned on.

Optionally, the termination instruction for the first pulse sequence includes an instruction outputted by the control unit 200 to control the second switching device to be turned off.

Optionally, as shown in Fig. 3, the second pulse generating circuit 120 further includes at least one fifth diode. An anode of the fifth diode is electrically connected to a second power source UL, and a cathode of the fifth diode is electrically connected to a first terminal of a second capacitor in a first level of second pulse generating unit 121.

Optionally, as shown in Fig. 3, the second pulse generating circuit 120 further includes at least one sixth diode, an anode and a cathode of the sixth diode are electrically connected to two adjacent second pulse generating units 121 respectively. The anode and the cathode of the third diode are electrically connected to a first terminal of a second switching device in a previous level of second pulse generating unit and a first terminal of a second capacitor in a next level of second pulse generating unit.

Optionally, as shown in Fig. 3, the pulse monitoring device 10 further includes a first capacitor discharge relay 112 and a second capacitor discharge relay 122. First terminals of the first capacitor discharge relay 112 and the second capacitor discharge relay 122 are both grounded, second terminals of the first capacitor discharge relay 112 and the second capacitor discharge relay 122 are electrically connected to a last level of first pulse generating unit 111 and a last level of second pulse generating unit 121 respectively.

Through the first capacitor discharge relay 112 and the second capacitor discharge relay 122, it is able to manually control the capacitors to discharge in special circumstances where rapid discharge is required, so as to ensure the capacitors in the pulse generating circuit 100 to discharge in a more thorough and faster manner.

Optionally, as shown in Fig. 3, the pulse monitoring device 10 further includes a first output relay 150, a second output relay 160 and a foot switch 170. A first terminal of the first output relay 150 is electrically connected to the first pulse generating circuit 110, the second pulse generating circuit 120, a second terminal of the first output relay 150 is electrically connected to a first terminal of the second output relay 160, and a second terminal of the second output relay 160 is electrically connected to a load Rload which is grounded. The foot switch 170 is electrically connected to a third terminal of the first output relay 150 and configured to control the load RLoad, the first output relay 150 and the first pulse generating circuit 110 or the second pulse generating circuit 120 to form a discharge circuit.

Through the first output relay 150, the second output relay 160 and the foot switch 170, it is able to enable a power supply circuit where the load RLoad (i.e., a human body part) is located to be opened, thereby improving safety. The second output relay 160 may be a 12-channel relay. The load RLoad is considered as an equivalent load of the target biological tissue.

Optionally, as shown in Fig. 3, the monitoring unit 300 includes a first Pearson coil 310, a second Pearson coil 320 and a first resistor R1. A first terminal of the first Pearson coil 310 is connected to the first resistor R1, the first resistor R1 is grounded, a second terminal of the first Pearson coil 310 is connected to a first terminal of the second Pearson coil 320, and a second terminal of the second Pearson coil 320 is electrically connected to the first pulse generating circuit 110 and the second pulse generating circuit 110. The feedback circuit is a circuit formed by the second pulse generating circuit 120 and the load RLoad.

Optionally, the first Pearson coil 310 has following functions. Firstly, it functions as a discharge channel for the pulses generated when the discharge circuit is not discharging. Secondly, a resistance of the first Pearson coil approximately ranges from 10kΩ to 100kΩ, it may be used for real-time voltage monitoring. The second Pearson coil 320 is used to measure current.

Optionally, when the second pulse generating circuit 120 is electrically connected to the load RLoad to form the feedback circuit, the first Pearson coil 310 and the second Pearson coil 320 serve as two sampling points for collecting real-time voltage and real-time current on the feedback circuit.

As an example, Fig. 3 shows a schematic diagram of the electrical connection between the pulse generation circuit 100 and the power source, the load RLoad as well as the monitoring unit. A power supply circuit where the first power source UH is located is a high-voltage nanosecond pulse generation circuit. A power supply circuit where the second power source UL is located is a low-voltage microsecond pulse generating circuit, and serves as a feedback circuit. The power supply circuit where the second power supply UL is located includes two levels of second pulse generating units 121, and outputs a voltage with an amplitude ranging from 0 to 500V. The power supply circuit where the first power supply UH is located may include twenty levels of first pulse generating units 111, and is capable of generating a high-voltage nanosecond pulse ranging from 0 to 15kV.

Optionally, in this embodiment, in the low-voltage microsecond pulse generating circuit, a capacitor C1, a switching device T1 and a diode D1 form one second pulse generating unit 121, and a capacitor C2, a switching device T2 and a diode D2 form another one second pulse generating unit 121. A diode D7 serves as the sixth diode, and a diode D6 serves as the fifth diode.

Optionally, in this embodiment, in the high-voltage nanosecond pulse generating circuit, a capacitor C3, a switching device T3 and a diode D3 form one first pulse generating unit 111, a capacitor C4, a switching device T4 and a diode D4 form another one first pulse generating unit 111, and a capacitor Cn, a switching device Tn and a diode Dn form yet another one first pulse generating unit 111. A diode D8, a diode D9, a diode D10, a diode D11 and a diode D12 are each the second diode, the diode D9, the diode D10, the diode D11 and the diode D12 are connected in series sequentially, and a diode D13 is the third diode.

Optionally, the switching device used in the low-voltage microsecond pulse generating circuit is a Metal-Oxide-Semiconductor Field-Effect Transistor (MOSFET), and the switching device used in the high-voltage nanosecond pulse generating circuit is an Insulated Gate Bipolar Transistor (IGBT).

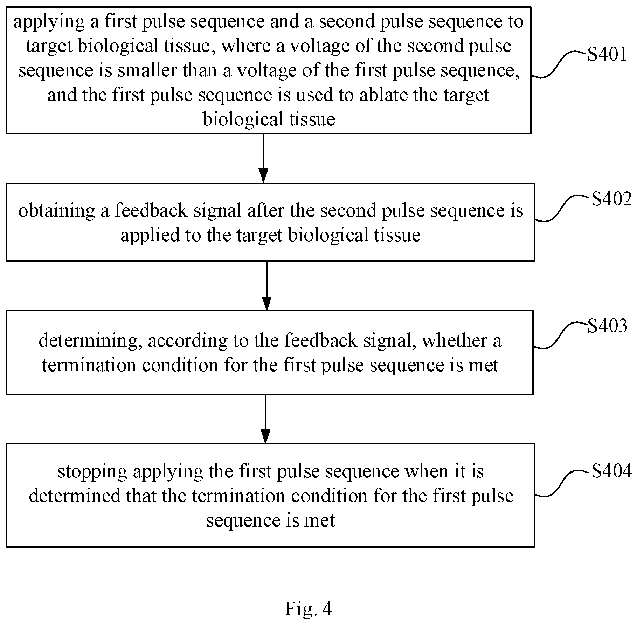

Based on the same inventive concept, the embodiments of the present application provide a pulse monitoring method, which is not in accordance with the appended claims and given as an example useful for the understanding of the invention. As shown in Fig. 4, the pulse monitoring method includes steps S401 to S404.

S401, applying a first pulse sequence and a second pulse sequence to target biological tissue, where a voltage of the second pulse sequence is smaller than a voltage of the first pulse sequence, and the first pulse sequence is used to ablate the target biological tissue.

In the embodiments of the present application, the second pulse sequence applied to the target biological tissue is used to reflect the status of the target biological tissue in real time and monitor the ablation status of the target biological tissue during the pulse ablation process, thereby guiding the pulse ablation process according to the ablation status of the target biological tissue.

Optionally, the pulse generating circuit 100 applies the first pulse sequence and the second pulse sequence to the target biological tissue.

In some embodiments of the present application, applying the first pulse sequence and the second pulse sequence to the target biological tissue includes applying a first set quantity of first pulse sequences and a second set quantity of second pulse sequences to the target biological tissue sequentially and alternately.

Optionally, the pulse generating circuit 100 sequentially and alternately applies the first set quantity of first pulse sequences and the second set quantity of second pulse sequences to the target biological tissue.

Optionally, the first set quantity and the second set quantity are the same or different. For example, when the first set quantity and the second set quantity are both 1, one first pulse sequence and one second pulse sequence are applied alternately. For another example, the first set quantity is 3, and the second set quantity is 1. After applying 3 first pulse sequences, 1 second pulse sequence is applied, which are performed in a repeated cycle.

In the embodiments of the present application, it is able to alternately apply the first pulse sequence and the second pulse sequence, so that the second pulse sequence is applied in each period, and the ablation status of the target biological tissue is monitored in real time, thereby to make an immediate response to the changes in the target biological tissue.

In some embodiments of the present application, the first pulse sequence includes at least one type of pulse and the second pulse sequence comprises at least one type of pulse.

Optionally, the first pulse sequence has only one type of pulse, and the second pulse sequence has only one type of pulse, and a voltage of the pulse in the first pulse sequence is higher than a voltage of the pulse in the second pulse sequence.

In some embodiments of the present application, the first pulse sequence includes a nanosecond pulse, or the first pulse sequence includes a nanosecond pulse and a microsecond pulse. The second pulse sequence includes a microsecond pulse.

Optionally, the first pulse sequence includes a nanosecond pulse and a microsecond pulse, and the microsecond pulse is also used to ablate the target biological tissue.

In some embodiments of the present application, the voltage of the first pulse sequence is greater than 500V and not greater than 15kV; and/or, the voltage of the second pulse sequence is not greater than 500V.

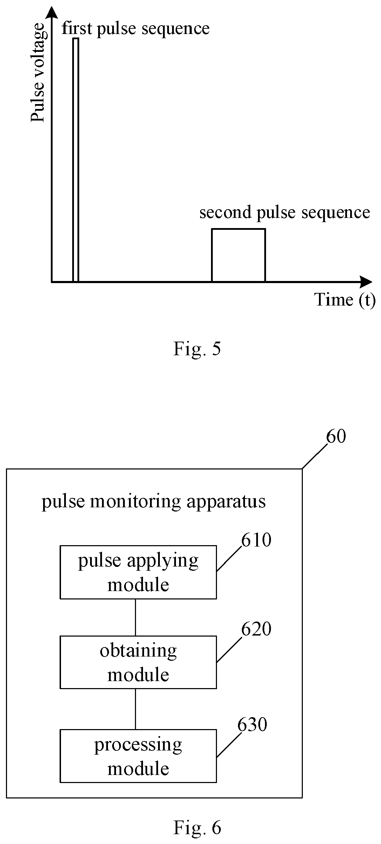

As an example, refer to Fig. 5, where the horizontal axis represents time and the vertical axis represents pulse voltage. A first pulse sequence is a high-voltage nanosecond pulse, a second pulse sequence is a low-voltage microsecond pulse, and the high-voltage nanosecond pulse and the low-voltage microsecond pulse are applied alternately. The high-voltage nanosecond pulse is used to ablate the target biological tissue, and the low-voltage microsecond pulse is used to monitor the ablation status of the target biological tissue.

Optionally, a current range of the high-voltage nanosecond pulse is 0 to 300A, and a pulse duration of the high-voltage nanosecond pulse is 200 nanoseconds to 1000 nanoseconds. A current range of the low-voltage microsecond pulse is 0 to100A, and a pulse duration of the low-voltage microsecond pulse is 10 microseconds to 300 microseconds.

S402, obtaining a feedback signal after the second pulse sequence is applied to the target biological tissue.

Optionally, the monitoring unit 300 obtains the feedback signal after the second pulse sequence is applied to the target biological tissue, and outputs the feedback signal to the control unit 200.

**In** some embodiments of the present application, obtaining the feedback signal after the second pulse sequence is applied to the target biological tissue includes: obtaining a real-time voltage and a real-time current of a feedback circuit corresponding to the target biological tissue after the second pulse sequence is applied to the target biological tissue.

S403, determining, according to the feedback signal, whether a termination condition for the first pulse sequence is met.

Optionally, the monitoring unit 300 outputs the feedback signal to the control unit 200, and the control unit 200 determines whether the termination condition for the first pulse sequence is met based on the feedback signal.

Through research, it has been found that, in the biological tissue, where cells are neatly and densely arranged, a single cell may be regarded as a basic structural unit with a specific impedance. Following the ablation with the high-voltage nanosecond pulse, some cells rupture, inevitably leading to changes in the impedance between two electrodes. Generally, the higher the degree of tissue ablation, the lower the impedance value. Therefore, the impedance may reflect the extent of tissue ablation to a certain degree.

Based on the aforementioned considerations, in some embodiments, determining whether the termination condition for the first pulse sequence is met according to the feedback signal includes:

determining, according to the real-time voltage and real-time current of the feedback circuit, a real-time impedance value of the target biological tissue;

determining whether the real-time impedance value is less than a set impedance value; or, displaying the real-time impedance value and determining whether a termination instruction for the first pulse sequence is received.

**In** some embodiments, determining whether the termination condition for the first pulse sequence is met according to the feedback signal includes:

determining, by the control unit 200, a real-time impedance value of the target biological tissue according to the real-time voltage and real-time current of the feedback circuit;

determining, by the control unit 200, whether the real-time impedance value is less than a set impedance value; or, displaying, by the display unit 400, the real-time impedance value and determining, by the control unit 200, whether a termination instruction for the first pulse sequence is received.

Optionally, the set impedance value is a value obtained by medical personnel based on ablation tests. When the real-time impedance value is less than the set impedance value, it is determined that an ablation expectation has been met.

Optionally, the process for calculating the real-time impedance value is shown in Equation (1):

$$Z = \frac{U_{in}}{I_m} \qquad \text{Equation (1)}$$

Uin represents a real-time voltage of the input pulse, Im denotes a real-time current, and Z is a calculated real-time impedance value. The calculated real-time impedance value serves as the equivalent impedance of the target biological tissue, which may accurately reflect the ablation status of the target biological tissue.

In some embodiments of the present application, displaying the real-time impedance value includes displaying a real-time impedance value curve, where the real-time impedance value curve includes at least two real-time impedance values in a set time period.

Optionally, displaying the real-time impedance value includes: displaying, by the display unit 400, a real-time impedance value curve, where the real-time impedance value curve includes at least two real-time impedance values in a set time period.

Optionally, the real-time impedance value is displayed to facilitate a doctor to determine whether to stop applying the first pulse sequence for ablation based on the real-time impedance value. In practical applications, the doctor may determine, based on experiences in combination with a current condition of the biological tissue, whether the ablation should continue or be terminated. When the real-time impedance value is displayed, it makes the entire medical process form a closed loop, and the doctor's decision-making can be based on evidence, so as to improve the ablation effect.

Optionally, displaying the real-time impedance value includes: displaying the real-time impedance value and corresponding biological indicator information of the target biological tissue, where the biological indicator information comprises at least one of the following: heart rate, blood pressure or blood oxygen concentration.

Optionally, displaying the real-time impedance value includes: displaying, by the display unit 400, the real-time impedance value and corresponding biological indicator information of the target biological tissue, where the biological indicator information comprises at least one of the following: heart rate, blood pressure or blood oxygen concentration.

Optionally, displaying the real-time impedance value and determining whether the termination instruction for the first pulse sequence is received includes: issuing an alarm prompt when the biological indicator information of the target biological tissue is greater than a set threshold, where the alarm prompt includes emitting an alarm sound and/or outputting alarm information. When receiving the alarm information, the control unit 200 outputs the termination instruction for the first pulse sequence.

Optionally, displaying the real-time impedance value and determining whether the termination instruction for the first pulse sequence is received includes: issuing, by the alarm unit 500, an alarm prompt when the biological indicator information of the target biological tissue is greater than a set threshold.

In the embodiments of the present application, it is able to simultaneously monitor the patient's biometric information while ablating the target biological tissue, so as to avoid the occurrence of danger during the ablation process of the patient, thereby to further ensure the ablation effect.

S404, stopping applying the first pulse sequence when it is determined that the termination condition for the first pulse sequence is met.

Optionally, stopping applying the first pulse sequence by the control unit 200 when it is determined that the termination condition for the first pulse sequence is met includes: stopping applying the first pulse sequence when it is determined that the real-time impedance value is less than the set impedance value; or, stopping applying the first pulse sequence when it is determined that the termination instruction for the first pulse sequence is received.

Optionally, stopping applying the first pulse sequence when it is determined that the termination instruction for the first pulse sequence is received includes: outputting, by the control unit 200, the termination instruction for the first pulse sequence to the pulse generating circuit 100 when determining that the real-time impedance value is less than the design impedance value, and controlling the pulse generating circuit 100 to stop applying the first pulse sequence; or, controlling, by the control unit 200, the pulse generating circuit 100 to stop applying the first pulse sequence when determining that the termination instruction for the first pulse sequence is received.

Optionally, when it is determined that the real-time impedance value is not less than the set impedance value, applying the first pulse sequence for ablation continues until the real-time impedance value is less than the set impedance value. When it is determined that the real-time impedance value is less than the set impedance value, it indicates that the ablation expectation is achieved, and it may continue to apply the first pulse sequence to ablate the target biological tissue.

In the above technical solutions of the embodiments of the present application, the target biological tissue is considered as an electrical network, the second pulse sequence is applied to the target biological tissue, and a response of the target biological tissue to this stimulus can be measured in the feedback circuit. Based on the stimulus and response, the equivalent real-time impedance value of the biological tissue can be determined, and the pulse ablation effect can be evaluated based on the real-time impedance value.

In the embodiments of the present application, it is able to calculate the real-time impedance value of the target biological tissue based on the real-time voltage and the real-time current, and based on whether a single pulse ablation, as indicated by the real-time impedance value, reaches the expected degree of tissue ablation, so as to guide medical personnel in the pulse ablation process.

Based on the same inventive concept, the embodiments of the present application provide a pulse monitoring apparatus, which is not in accordance with the appended claims and given as an example useful for the understanding of the invention. As shown in Fig. 6, the pulse monitoring apparatus 60 includes a pulse applying module 610, an obtaining module 620 and a processing module 630.

The pulse applying module 610 is configured to apply a first pulse sequence and a second pulse sequence to target biological tissue, where a voltage of the second pulse sequence is smaller than a voltage of the first pulse sequence, and the first pulse sequence is used to ablate the target biological tissue.

The obtaining module 620 is configured to obtain a feedback signal after the second pulse sequence is applied to the target biological tissue.

The processing module 630 is configured to determine, according to the feedback signal, whether a termination condition for the first pulse sequence is met, and stop applying the first pulse sequence when it is determined that the termination condition for the first pulse sequence is met.

Optionally, the pulse applying module 610 is further configured to apply a first set quantity of first pulse sequences and a second set quantity of second pulse sequences to the target biological tissue sequentially and alternately.

Optionally, the obtaining module 620 is further configured to obtain a real-time voltage and a real-time current of a feedback circuit corresponding to the target biological tissue after the second pulse sequence is applied to the target biological tissue.

Optionally, the processing module 630 is further configured to determine, according to the real-time voltage and real-time current of the feedback circuit, a real-time impedance value of the target biological tissue; determine whether the real-time impedance value is less than a set impedance value; or, display the real-time impedance value and determine whether a termination instruction for the first pulse sequence is received.

Optionally, the processing module 630 is further configured to display a real-time impedance value curve, where the real-time impedance value curve includes at least two real-time impedance values in a set time period, or, display the real-time impedance value and corresponding biological indicator information of the target biological tissue, where the biological indicator information includes at least one of the following: heart rate, blood pressure or blood oxygen concentration.

Optionally, the processing module 630 is further configured to stop applying the first pulse sequence when it is determined that the real-time impedance value is less than the set impedance value, or, stop applying the first pulse sequence when it is determined that the termination instruction for the first pulse sequence is received.

Based on the same inventive concept, the embodiments of the present application provide a computer-readable storage medium having a computer program stored thereon, the computer program implements, when executed by the pulse monitoring device 10, the pulse monitoring method in any embodiment of the present application.

The computer-readable medium in the embodiments of the present application may be a computer-readable signal medium or a computer-readable storage medium, or any combination thereof. The computer-readable storage medium may be, for example, but not limited to, an electronic, magnetic, optical, electromagnetic, infrared, or semiconductor system, apparatus or device, or any combination thereof. In more specific examples, the computer readable storage medium may include, but not limited to, an electrical connection having one or more wires, a portable computer disk, a hard drive, a random access memory (RAM), a read only memory (ROM), a removable programmable read-only memory (EPROM or flash memory), an optical fiber, a portable compact disk read-only memory (CD-ROM), an optical storage device, a magnetic storage device, or any suitable combination thereof.

The computer-readable medium in the embodiments of the present application may be any tangible medium containing or storing a program, and the program may be used by or in combination with an instruction execution system, apparatus or device. In the present application, the computer-readable signal medium may include a data signal in baseband or propagated as part of a carrier wave, in which computer-readable program code is carried. The propagated data signal may take many forms, including but not limited to an electromagnetic signal, an optical signal, or any suitable combination thereof. The computer-readable signal medium may also be any computer-readable medium other than the computer-readable storage medium that can send, propagate, or transmit a program used by or in connection with an instruction execution system, apparatus, or device. Program code on the computer-readable medium may be transmitted by using any suitable medium, including but not limited to: wire, optical fiber cable, radio frequency (RF), etc., or any suitable combination thereof.

When the embodiments of the present application are implemented, at least the following beneficial effects can be achieved.

(1) In the pulse monitoring method of the embodiments of the present application, during the pulse ablation process, it is able to determine an ablation status of the target biological tissue based on the feedback signal, so as to monitor the ablation status of the target biological tissue during the pulse ablation process in real time. When it is determined that a termination condition for the first pulse sequence is met, the output of the first pulse sequence is stopped. That is, in the embodiments of the present application, it is able to guide the pulse ablation process according to changes in the biological tissue. After determining that the pulse ablation effect is achieved, the output of the first pulse sequence for ablating the biological tissue is stopped, thereby to guide the pulse ablation process according to the changes in the target biological tissue.

(2) In the embodiments of the present application, it is able to alternately apply the first pulse sequence and the second pulse sequence, so that the second pulse sequence is applied in each period, and the ablation status of the target biological tissue is monitored in real time, thereby to make an immediate response to the changes in the target biological tissue.

(3) In the embodiments of the present application, it is able to simultaneously monitor the patient's biometric information while ablating the target biological tissue, so as to avoid the occurrence of danger during the ablation process of the patient, thereby to further ensure the ablation effect.

(4) In the embodiments of the present application, it is able to calculate the real-time impedance value of the target biological tissue based on the real-time voltage and the real-time current, and based on whether a single pulse ablation, as indicated by the real-time impedance value, reaches the expected degree of tissue ablation, so as to guide medical personnel in the pulse ablation process.

(5) In the embodiments of the present application, the first pulse generating circuit 110 and the second pulse generating circuit 120 are integrated on the same circuit board, so it allows a single circuit board to output the first pulse sequence and the second pulse sequence, accommodating both ablation and monitoring functionalities.

(6) Through the pulse monitoring device 10 in the embodiments of the present application, it realizes that two functions of pulse ablation and monitoring are integrated into one device, and thereby it does not need to be equipped with other monitoring devices or independent modules, making it convenient to use and operate.

As can be appreciated by a person skilled in the art, steps, measures and schemes in various operations, methods and processes that have already been discussed in the embodiments of the present application may be replaced, modified, combined or deleted. In a possible embodiment of the present disclosure, the other steps, measures and schemes in various operations, methods and processes that have already been discussed in the embodiments of the present application may also be replaced, modified, rearranged, decomposed, combined or deleted. In another possible embodiment of the present application, steps, measures and schemes in various operations, methods and processes that are known in the related art and have already been discussed in the embodiments of the present application may also be replaced, modified, rearranged, decomposed, combined or deleted.

Such words as "first" and "second" are merely for illustrative purposes, rather than to implicitly or explicitly indicate the number of the defined technical features. In this regard, the technical features defined with such words as "first" and "second" may implicitly or explicitly include one or more technical features. Further, such a phrase as "a plurality of" is used to indicate that there are at least two, e.g., two or three, components, unless otherwise specified.

It should be further appreciated that, although with arrows, the steps in the flow charts may not be necessarily performed in an order indicated by the arrows. Unless otherwise defined, the order of the steps may not be strictly defined, i.e., the steps may also be performed in another order. In addition, each of at least parts of the steps in the flow charts may include a plurality of sub-steps or stages, and these sub-steps or stages may not be necessarily performed at the same time, i.e., they may also be performed at different times. Furthermore, these sub-steps or stages may not be necessarily performed sequentially, and instead, they may be performed alternately with the other steps or at least parts of sub-steps or stages of the other steps.

The above embodiments are partial embodiments of the present application, it should be appreciated that those skilled in the art may make various improvements and modifications without departing from the principle of the present disclosure, and theses improvement and modifications shall fall within the scope of the present disclosure.

## Claims

1.  A computer-readable storage medium having a computer program stored thereon, wherein the computer program implements, when executed by a pulse monitoring device, the following:

     applying (S401) a first pulse sequence and a second pulse sequence to target biological tissue; wherein a voltage of the second pulse sequence is smaller than a voltage of the first pulse sequence, and the first pulse sequence is used to ablate the target biological tissue;
     obtaining (S402) a feedback signal after the second pulse sequence is applied to the target biological tissue;
     determining (S403), according to the feedback signal, whether a termination condition for the first pulse sequence is met;
     stopping applying (S404) the first pulse sequence when it is determined that the termination condition for the first pulse sequence is met;
     wherein the voltage of the first pulse sequence is greater than 500V and not greater than 15kV; and/or,
     the voltage of the second pulse sequence is not greater than 500V;
     wherein obtaining the feedback signal after the second pulse sequence is applied to the target biological tissue comprises:

       obtaining a real-time voltage and a real-time current of a feedback circuit corresponding to the target biological tissue after the second pulse sequence is applied to the target biological tissue;
       wherein determining whether the termination condition for the first pulse sequence is met according to the feedback signal comprises:

         determining, according to the real-time voltage and real-time current of the feedback circuit, a real-time impedance value of the target biological tissue; and
         determining whether the real-time impedance value is less than a set impedance value; or, displaying the real-time impedance value and determining whether a termination instruction for the first pulse sequence is received; and

wherein stopping applying the first pulse sequence when it is determined that the termination condition for the first pulse sequence is met comprises:

stopping applying the first pulse sequence when it is determined that the real-time impedance value is less than the set impedance value; or, stopping applying the first pulse sequence when it is determined that the termination instruction for the first pulse sequence is received.

2. The computer-readable storage medium according to claim 1, wherein applying the first pulse sequence and the second pulse sequence to the target biological tissue comprises:
applying a first set quantity of first pulse sequences and a second set quantity of second pulse sequences to the target biological tissue sequentially and alternately.

3. The computer-readable storage medium according to claim 1 or 2, wherein the first pulse sequence comprises at least one type of pulse and the second pulse sequence comprises at least one type of pulse.

4. The computer-readable storage medium according to any one of claims 1 to 3, wherein the first pulse sequence comprises a nanosecond pulse, or the first pulse sequence comprises a nanosecond pulse and a microsecond pulse; the second pulse sequence comprises a microsecond pulse.

5. The computer-readable storage medium according to claim 1, wherein displaying the real-time impedance value comprises at least one of the following:

displaying a real-time impedance value curve, wherein the real-time impedance value curve comprises at least two real-time impedance values in a set time period; or,
displaying the real-time impedance value and corresponding biological indicator information of the target biological tissue; wherein the biological indicator information comprises at least one of the following: heart rate, blood pressure or blood oxygen concentration.

6. The computer-readable storage medium according to claim 1 or 5, wherein displaying the real-time impedance value and determining whether the termination instruction for the first pulse sequence is received comprises:

issuing an alarm prompt when the biological indicator information of the target biological tissue is greater than a set threshold, wherein the alarm prompt comprises emitting an alarm sound and/or outputting alarm information;
outputting the termination instruction for the first pulse sequence in response to receiving the alarm information.

7. A pulse monitoring device (10), comprising: a pulse generating circuit (100), a control unit (200) and a monitoring unit (300); wherein,

the control unit (200) is communicatively connected to the pulse generating circuit (100), and configured to control the pulse generating circuit (100) to apply a first pulse sequence and a second pulse sequence to target biological tissue; wherein a voltage of the second pulse sequence is smaller than a voltage of the first pulse sequence, and the first pulse sequence is used to ablate the target biological tissue;
the control unit (200) is further configured to determine, according to a feedback signal forwarded by the monitoring unit (300), whether a termination condition for the first pulse sequence is met, and output a termination instruction for the first pulse sequence to the pulse generating circuit (100) when it is determined that the termination condition for the first pulse sequence is met;
the monitoring unit (300) is communicatively connected to the control unit (200), and configured to obtain the feedback signal after the second pulse sequence is applied to the target biological tissue, and output the feedback signal to the control unit (200);
wherein the monitoring unit (300) is further configured to obtain a real-time voltage and a real-time current of a feedback circuit corresponding to the target biological tissue after the second pulse sequence is applied to the target biological tissue;
wherein the control unit (200) is further configured to:

determine, according to the real-time voltage and real-time current of the feedback circuit, a real-time impedance value of the target biological tissue; and
determine whether the real-time impedance value is less than a set impedance value; or, display through a display unit (400) the real-time impedance value and determine whether the termination instruction for the first pulse sequence is received; and

wherein the control unit (200) is further configured to:

stop applying the first pulse sequence when it is determined that the real-time impedance value is less than the set impedance value; or, stop applying the first pulse sequence when it is determined that the termination instruction for the first pulse sequence is received;

**characterized in that** the pulse generating circuit (100) comprises a first pulse generating circuit (110) for outputting the first pulse sequence and a second pulse generating circuit (120) for outputting the second pulse sequence;

wherein the first pulse generating circuit (110) and the second pulse generating circuit (120) are integrated on a same circuit board;

wherein the first pulse generating circuit (110) comprises at least one level of first pulse generating units (111) electrically connected sequentially;

wherein each first pulse generating unit (111) is electrically connected to the control unit (200), and configured to be turned on under control of the control unit (200) and apply the first pulse sequence to the target biological tissue;

wherein each of the at least one level of first pulse generating units (111) comprises a first capacitor, a first switching device and a first diode, a first terminal of the first capacitor is electrically connected to a first terminal of the first switching device, a positive electrode and a negative electrode of the first diode are electrically connected to a second terminal of the first capacitor and a second terminal of the first switching device respectively, and a control terminal of the first switching device is electrically connected to the control unit (200).

8. The pulse monitoring device (10) according to claim 7, wherein the first pulse generating circuit (110) further comprises at least one second diode; a first terminal of a first capacitor in a first level of first pulse generating unit (111) in the at least one level of first pulse generating units (111) is electrically connected to a first power source through the at least one second diode.

9. The pulse monitoring device (10) according to claim 7 or 8, wherein the first pulse generating circuit (110) further comprises at least one third diode, an anode and a cathode of the third diode are electrically connected to two adjacent first pulse generating units (111) respectively.

10. The pulse monitoring device (10) according to any one of claims 7 to 9, wherein the second pulse generating circuit (120) comprises at least one level of second pulse generating units (121) electrically connected sequentially; wherein each second pulse generating unit (121) is electrically connected to the control unit (200) and configured to be turned on under control of the control unit (200) and apply the second pulse sequence to the target biological tissue.

11. The pulse monitoring device (10) according to claim 10, wherein the second pulse generating unit (121) comprises a second capacitor, a second switching device and a fourth diode; a first terminal of the second capacitor is electrically connected to a first terminal of the second switching device, a positive electrode and a negative electrode of the fourth diode are electrically connected to a second terminal of the second capacitor and a second terminal of the second switching device respectively, and a control terminal of the second switching device is electrically connected to the control unit (200).

12. The pulse monitoring device (10) according to any one of claims 7 to 10, wherein the display unit (400) is communicatively connected to the control unit (200), wherein the display unit (400) is configured to display at least one of a real-time impedance value, a real-time impedance value curve or biological indicator information.

13. The pulse monitoring device (10) according to any one of claims 7 to 12, wherein the pulse monitoring device further comprises an alarm unit (500) communicatively connected to the control unit (200), wherein the alarm unit (500) is configured to issue an alarm prompt when the biological indicator information of the target biological tissue is greater than a set threshold.

**Patentansprüche**

1. Computerlesbares Speichermedium, aufweisend ein darauf gespeichertes Computerprogramm, wobei das Computerprogramm bei Ausführung durch eine Impulsüberwachungsvorrichtung Folgendes implementiert:

Anwenden (S401) einer ersten Impulssequenz und einer zweiten Impulssequenz auf biologisches Zielgewebe, wobei eine Spannung der zweiten Impulssequenz kleiner ist als eine Spannung der ersten Impulssequenz und die erste Impulssequenz genutzt wird, um das biologische Zielgewebe zu ablatieren;

Erlangen (S402) eines Feedbacksignals, nachdem die zweite Impulssequenz auf das biologische Zielgewebe angewandt wurde;

Bestimmen (S403) gemäß dem Feedbacksignal, ob eine Beendigungsbedingung für die erste Impulssequenz erfüllt ist;

Stoppen des Anwendens (S404) der ersten Impulssequenz, wenn bestimmt wird, dass die Beendigungsbedingung für die erste Impulssequenz erfüllt ist,

wobei die Spannung der ersten Impulssequenz größer als 500 V und nicht größer als 15 kV ist und/oder die Spannung der zweiten Impulssequenz nicht größer als 500 V ist,

wobei das Erlangen des Feedbacksignals, nachdem die zweite Impulssequenz auf das biologische Zielgewebe angewandt wurde, Folgendes umfasst:

Erlangen einer Echtzeitspannung und eines Echtzeitstroms einer Feedbackschaltung entsprechend dem biologischen Zielgewebe, nachdem die zweite Impulssequenz auf das biologische Zielgewebe angewandt wurde,

wobei das Bestimmen gemäß dem Feedbacksignal, ob die Beendigungsbedingung für die erste Impulssequenz erfüllt ist, Folgendes umfasst:

Bestimmen eines Echtzeitimpedanzwerts des biologischen Zielgewebes gemäß der Echtzeitspannung und dem Echtzeitstrom der Feedbackschaltung und

Bestimmen, ob der Echtzeitimpedanzwert kleiner ist als ein festgelegter Impedanzwert, oder Anzeigen des Echtzeitimpedanzwerts und Bestimmen, ob eine Beendigungsanweisung für die erste Impulssequenz eingegangen ist, und

wobei das Stoppen des Anwendens der ersten Impulssequenz, wenn bestimmt wird, dass die Beendigungsbedingung für die erste Impulssequenz erfüllt ist, Folgendes umfasst:

Stoppen des Anwendens der ersten Impulssequenz, wenn bestimmt wird, dass der Echtzeitimpedanzwert kleiner ist als der festgelegte Impedanzwert, oder Stoppen des Anwendens der ersten Impulssequenz, wenn bestimmt wird, dass die Beendigungsanweisung für die erste Impulssequenz eingegangen ist.

2. Computerlesbares Speichermedium nach Anspruch 1, wobei das Anwenden der ersten Impulssequenz und der zweiten Impulssequenz auf das biologische Zielgewebe Folgendes umfasst:
Anwenden einer ersten festgelegten Menge von ersten Impulssequenzen und einer zweiten festgelegten Menge von zweiten Impulssequenzen auf das biologische Zielgewebe in sequenzieller und abwechselnder Weise.

3. Computerlesbares Speichermedium nach Anspruch 1 oder 2, wobei die erste Impulssequenz mindestens einen Impulstyp umfasst und die zweite Impulssequenz mindestens einen Impulstyp umfasst.

4. Computerlesbares Speichermedium nach einem der Ansprüche 1 bis 3, wobei die erste Impulssequenz einen Nanosekundenimpuls umfasst oder die erste Impulssequenz einen Nanosekundenimpuls und einen Mikrosekundenimpuls umfasst,
wobei die zweite Impulssequenz einen Mikrosekundenimpuls umfasst.

5. Computerlesbares Speichermedium nach Anspruch 1, wobei das Anzeigen des Echtzeitimpedanzwerts mindestens einen der folgenden Vorgänge umfasst:

Anzeigen einer Echtzeitimpedanzwertkurve, wobei die Echtzeitimpedanzwertkurve mindestens zwei Echtzeitimpedanzwerte in einem festgelegten Zeitraum umfasst, oder
Anzeigen des Echtzeitimpedanzwerts und entsprechender biologischer Indikatorinformationen des biologischen Zielgewebes, wobei die biologischen Indikatorinformationen mindestens einen der folgenden Werte umfassen: Herzfrequenz, Blutdruck oder Sauerstoffkonzentration im Blut.

6. Computerlesbares Speichermedium nach Anspruch 1 oder 5, wobei das Anzeigen des Echtzeitimpedanzwerts und das Bestimmen, ob die Beendigungsanweisung für die erste Impulssequenz eingegangen ist, Folgendes umfassen:

Auslösen einer Alarmmeldung, wenn die biologischen Indikatorinformationen des biologischen Zielgewebes größer sind als ein festgelegter Schwellenwert, wobei die Alarmmeldung das Aussenden eines Alarmtons und/oder das Ausgeben von Alarminformationen umfasst;

Ausgeben der Beendigungsanweisung für die erste Impulssequenz als Reaktion auf das Eingehen der Alarminformationen.

**7.** Impulsüberwachungsvorrichtung (10), umfassend: eine Impulserzeugungsschaltung (100), eine Steuereinheit (200) und eine Überwachungseinheit (300), wobei

die Steuereinheit (200) kommunikativ mit der Impulserzeugungsschaltung (100) verbunden und ausgelegt ist, um die Impulserzeugungsschaltung (100) zu steuern, um eine erste Impulssequenz und eine zweite Impulssequenz auf biologisches Zielgewebe anzuwenden, wobei eine Spannung der zweiten Impulssequenz kleiner ist als eine Spannung der ersten Impulssequenz und die erste Impulssequenz genutzt wird, um das biologische Zielgewebe zu ablatieren;

die Steuereinheit (200) ferner ausgelegt ist, um gemäß einem von der Überwachungseinheit (300) weitergeleiteten Feedbacksignal zu bestimmen, ob eine Beendigungsbedingung für die erste Impulssequenz erfüllt ist, und eine Beendigungsanweisung für die erste Impulssequenz an die Impulserzeugungsschaltung (100) auszugeben, wenn bestimmt wird, dass die Beendigungsbedingung für die erste Impulssequenz erfüllt ist;

die Überwachungseinheit (300) kommunikativ mit der Steuereinheit (200) verbunden und ausgelegt ist, um das Feedbacksignal zu erlangen, nachdem die zweite Impulssequenz auf das biologische Zielgewebe angewandt wurde, und das Feedbacksignal an die Steuereinheit (200) auszugeben,

wobei die Überwachungseinheit (300) ferner ausgelegt ist, um eine Echtzeitspannung und einen Echtzeitstrom einer Feedbackschaltung entsprechend dem biologischen Zielgewebe zu erlangen, nachdem die zweite Impulssequenz auf das biologische Zielgewebe angewandt wurde,

wobei die Steuereinheit (200) ferner ausgelegt ist, um:

einen Echtzeitimpedanzwert des biologischen Zielgewebes gemäß der Echtzeitspannung und dem Echtzeitstrom der Feedbackschaltung zu bestimmen und

zu bestimmen, ob der Echtzeitimpedanzwert kleiner ist als ein festgelegter Impedanzwert, oder den Echtzeitimpedanzwert durch eine Anzeigeeinheit (400) anzuzeigen und zu bestimmen, ob die Beendigungsanweisung für die erste Impulssequenz eingegangen ist, und

wobei die Steuereinheit (200) ferner ausgelegt ist, um:

das Anwenden der ersten Impulssequenz zu stoppen, wenn bestimmt wird, dass der Echtzeitimpedanzwert kleiner ist als der festgelegte Impedanzwert, oder das Anwenden der ersten Impulssequenz zu stoppen, wenn bestimmt wird, dass die Beendigungsanweisung für die erste Impulssequenz eingegangen ist,

**dadurch gekennzeichnet, dass** die Impulserzeugungsschaltung (100) eine erste Impulserzeugungsschaltung (110) umfasst, um die erste Impulssequenz auszugeben, und eine zweite Impulserzeugungsschaltung (120), um die zweite Impulssequenz auszugeben,

wobei die erste Impulserzeugungsschaltung (110) und die zweite Impulserzeugungsschaltung (120) auf einer selben Leiterplatte integriert sind,

wobei die erste Impulserzeugungsschaltung (110) mindestens eine Stufe von ersten Impulserzeugungseinheiten (111) umfasst, die nacheinander elektrisch verbunden sind,

wobei jede erste Impulserzeugungseinheit (111) elektrisch mit der Steuereinheit (200) verbunden und ausgelegt ist, um unter Steuerung der Steuereinheit (200) eingeschaltet zu werden und die erste Impulssequenz auf das biologische Zielgewebe anzuwenden,

wobei eine jede der mindestens einen Stufe von ersten Impulserzeugungseinheiten (111) einen ersten Kondensator, eine erste Schaltvorrichtung und eine erste Diode umfasst, ein erster Anschluss des ersten Kondensators elektrisch mit einem ersten Anschluss der ersten Schaltvorrichtung verbunden ist, eine positive Elektrode und eine negative Elektrode der ersten Diode elektrisch jeweils mit einem zweiten Anschluss des ersten Kondensators und einem zweiten Anschluss der ersten Schaltvorrichtung verbunden sind und ein Steueranschluss der ersten Schaltvorrichtung elektrisch mit der Steuereinheit (200) verbunden ist.

**8.** Impulsüberwachungsvorrichtung (10) nach Anspruch 7, wobei die erste Impulserzeugungsschaltung (110) ferner mindestens eine zweite Diode umfasst und ein erster Anschluss eines ersten Kondensators in einer ersten Stufe einer ersten Impulserzeugungseinheit (111) in der mindestens einen Stufe von ersten Impulserzeugungseinheiten (111) elektrisch mit einer ersten Stromquelle durch die mindestens eine zweite Diode verbunden ist.

9. Impulsüberwachungsvorrichtung (10) nach Anspruch 7 oder 8, wobei die erste Impulserzeugungsschaltung (110) ferner mindestens eine dritte Diode umfasst und eine Anode und eine Kathode der dritten Diode elektrisch jeweils mit zwei benachbarten ersten Impulserzeugungseinheiten (111) verbunden sind.

10. Impulsüberwachungsvorrichtung (10) nach einem der Ansprüche 7 bis 9, wobei die zweite Impulserzeugungsschaltung (120) mindestens eine Stufe von zweiten Impulserzeugungseinheiten (121) umfasst, die nacheinander elektrisch verbunden sind,
wobei jede zweite Impulserzeugungseinheit (121) elektrisch mit der Steuereinheit (200) verbunden und ausgelegt ist, um unter Steuerung der Steuereinheit (200) eingeschaltet zu werden und die zweite Impulssequenz auf das biologische Zielgewebe anzuwenden.

11. Impulsüberwachungsvorrichtung (10) nach Anspruch 10, wobei die zweite Impulserzeugungseinheit (121) einen zweiten Kondensator, eine zweite Schaltvorrichtung und eine vierte Diode umfasst, ein erster Anschluss des zweiten Kondensators elektrisch mit einem ersten Anschluss der zweiten Schaltvorrichtung verbunden ist, eine positive Elektrode und eine negative Elektrode der vierten Diode elektrisch jeweils mit einem zweiten Anschluss des zweiten Kondensators und einem zweiten Anschluss der zweiten Schaltvorrichtung verbunden sind und ein Steueranschluss der zweiten Schaltvorrichtung elektrisch mit der Steuereinheit (200) verbunden ist.

12. Impulsüberwachungsvorrichtung (10) nach einem der Ansprüche 7 bis 10, wobei die Anzeigeeinheit (400) kommunikativ mit der Steuereinheit (200) verbunden ist, wobei die Anzeigeeinheit (400) ausgelegt ist, um mindestens entweder einen Echtzeitimpedanzwert, eine Echtzeitimpedanzwertkurve oder biologische Indikatorinformationen anzuzeigen.

13. Impulsüberwachungsvorrichtung (10) nach einem der Ansprüche 7 bis 12, wobei die Impulsüberwachungsvorrichtung ferner eine Alarmeinheit (500) umfasst, die kommunikativ mit der Steuereinheit (200) verbunden ist, wobei die Alarmeinheit (500) ausgelegt ist, um eine Alarmmeldung auszulösen, wenn die biologischen Indikatorinformationen des biologischen Zielgewebes größer sind als ein festgelegter Schwellenwert.

## Revendications

1. Support de stockage lisible par ordinateur sur lequel est stocké un programme informatique, le programme informatique mettant en œuvre, lorsqu'il est exécuté par un dispositif de surveillance d'impulsions, les étapes suivantes :

   application (S401) d'une première séquence d'impulsions et d'une deuxième séquence d'impulsions à un tissu biologique cible ; une tension de la deuxième séquence d'impulsions étant inférieure à une tension de la première séquence d'impulsions, et la première séquence d'impulsions étant utilisée pour effectuer l'ablation du tissu biologique cible ;
   obtention (S402) d'un signal de rétroaction après l'application de la deuxième séquence d'impulsions au tissu biologique cible ;
   détermination (S403), selon le signal de rétroaction, si une condition de fin pour la première séquence d'impulsions est remplie ;
   arrêt de l'application (S404) de la première séquence d'impulsions lorsqu'il est déterminé que la condition de fin pour la première séquence d'impulsions est remplie ;
   la tension de la première séquence d'impulsions étant supérieure à 500 V et n'étant pas supérieure à 15 kV ; et/ou la tension de la deuxième séquence d'impulsions n'étant pas supérieure à 500 V ;
   l'obtention du signal de rétroaction après l'application de la deuxième séquence d'impulsions au tissu biologique cible comprenant ;
   l'obtention d'une tension en temps réel et d'un courant en temps réel d'un circuit de rétroaction correspondant au tissu biologique cible après l'application de la deuxième séquence d'impulsions au tissu biologique cible ;
   la détermination si la condition de fin pour la première séquence d'impulsions est remplie selon le signal de rétroaction comprenant :

   la détermination, selon la tension en temps réel et le courant en temps réel du circuit de rétroaction, d'une valeur d'impédance en temps réel du tissu biologique cible ; et
   la détermination si la valeur d'impédance en temps réel est inférieure à une valeur d'impédance définie ; ou
   l'affichage de la valeur d'impédance en temps réel et la détermination si une instruction de fin pour la première séquence d'impulsions est reçue ; et

l'arrêt de l'application de la première séquence d'impulsions lorsqu'il est déterminé que la condition de fin pour la première séquence d'impulsions est remplie comprenant ;

l'arrêt de l'application de la première séquence d'impulsions lorsqu'il est déterminé que la valeur d'impédance en temps réel est inférieure à la valeur d'impédance définie ; ou l'arrêt de l'application de la première séquence d'impulsions lorsqu'il est déterminé que l'instruction de fin pour la première séquence d'impulsions est reçue.

2. Support de stockage lisible par ordinateur selon la revendication 1, dans lequel l'application de la première séquence d'impulsions et de la deuxième séquence d'impulsions au tissu biologique cible comprend :

l'application séquentiellement et alternativement d'une première quantité définie de premières séquences d'impulsions et d'une deuxième quantité définie de deuxièmes séquences d'impulsions au tissu biologique cible.

3. Support de stockage lisible par ordinateur selon la revendication 1 ou 2, dans lequel la première séquence d'impulsions comprend au moins un type d'impulsions et la deuxième séquence d'impulsions comprend au moins un type d'impulsions.

4. Support de stockage lisible par ordinateur selon l'une quelconque des revendications 1 à 3, dans lequel la première séquence d'impulsions comprend une impulsion nanoseconde, ou la première séquence d'impulsions comprend une impulsion nanoseconde et une impulsion microseconde ;

la deuxième séquence d'impulsions comprend une impulsion microseconde.

5. Support de stockage lisible par ordinateur selon la revendication 1, dans lequel l'affichage de la valeur d'impédance en temps réel comprend au moins l'une des actions suivantes :

l'affichage d'une courbe de valeur d'impédance en temps réel, la courbe de valeur d'impédance en temps réel comprenant au moins deux valeurs d'impédance en temps réel dans une période de temps définie ; ou,

l'affichage de la valeur d'impédance en temps réel et d'informations d'indicateur biologique correspondantes du tissu biologique cible, les informations d'indicateur biologique comprenant au moins une des informations suivantes : fréquence cardiaque, pression artérielle ou concentration d'oxygène dans le sang.

6. Support de stockage lisible par ordinateur selon la revendication 1 ou 5, dans lequel l'affichage de la valeur d'impédance en temps réel et la détermination si l'instruction de fin pour la première séquence d'impulsions est reçue comprennent :

l'émission d'une invite d'alarme lorsque les informations d'indicateur biologique du tissu biologique cible sont supérieures à un seuil défini, l'invite d'alarme comprenant l'émission d'un son d'alarme et/ou la sortie d'informations d'alarme ;

la sortie d'une instruction de fin pour la première séquence d'impulsions en réponse à la réception des informations d'alarme.

7. Dispositif de surveillance d'impulsions (10) comprenant : un circuit générateur d'impulsions (100), une unité de commande (200) et une unité de surveillance (300) ; dans lequel

l'unité de commande (200) est connectée en communication au circuit générateur d'impulsions (100), et configurée pour commander le circuit générateur d'impulsions (100) à appliquer une première séquence d'impulsions et une deuxième séquence d'impulsions à un tissu biologique cible ; une tension de la deuxième séquence d'impulsions étant inférieure à une tension de la première séquence d'impulsions, et la première séquence d'impulsions étant utilisée pour effectuer l'ablation du tissu biologique cible ;

l'unité de commande (200) est en outre configurée pour déterminer, selon un signal de rétroaction transmis par l'unité de surveillance (300), si une condition de fin pour la première séquence d'impulsions est remplie, et pour sortir une instruction de fin pour la première séquence d'impulsions au circuit générateur d'impulsions (100) lorsqu'il est déterminé que la condition de fin pour la première séquence d'impulsions est remplie ;

L'unité de surveillance (300) est connectée en communication à l'unité de commande (200), et configurée pour obtenir le signal de rétroaction après l'application de la deuxième séquence d'impulsions au tissu biologique cible, et pour sortir le signal de rétroaction à l'unité de commande 200 ;

l'unité de surveillance (300) étant en outre configurée pour obtenir une tension en temps réel et un courant en temps réel d'un circuit de rétroaction correspondant au tissu biologique cible après l'application de la deuxième séquence d'impulsions au tissu biologique cible ;

l'unité de commande (200) étant en outre configurée pour :

déterminer, selon la tension en temps réel et le courant en temps réel du circuit de rétroaction, une valeur d'impédance en temps réel du tissu biologique cible ; et

déterminer si la valeur d'impédance en temps réel est inférieure à une valeur d'impédance définie ; ou afficher par le biais d'une unité d'affichage (400) la valeur d'impédance en temps réel et déterminer si l'instruction de fin pour la première séquence d'impulsions est reçue ; et

l'unité de commande (200) étant en outre configurée pour :

arrêter l'application de la première séquence d'impulsions lorsqu'il est déterminé que la valeur d'impédance en temps réel est inférieure à la valeur d'impédance définie ; ou arrêter l'application de la première séquence d'impulsions lorsqu'il est déterminé que l'instruction de fin pour la première séquence d'impulsions est reçue;

**caractérisé en ce que** le circuit générateur d'impulsions (100) comprend un premier circuit générateur d'impulsions (110) pour la sortie de la première séquence d'impulsions et un deuxième circuit générateur d'impulsions (120) pour la sortie de la deuxième séquence d'impulsions ;

le premier circuit générateur d'impulsions (110) et le deuxième circuit générateur d'impulsions (120) étant intégrés sur une même carte de circuit imprimé ;

le premier circuit générateur d'impulsions (110) comprenant au moins un niveau de premières unités de génération d'impulsions (111) connectées électriquement de manière séquentielle ;

chaque première unité de génération d'impulsions (111) étant connectée électriquement à l'unité de commande (200) et configurée pour être mise en marche sous la commande de l'unité de commande (200) et pour appliquer la première séquence d'impulsions au tissu biologique cible ;

chacun de l'au moins un niveau de premières unités de génération d'impulsions (111) comprenant un premier condensateur, un premier dispositif de commutation et une première diode, une première borne du premier condensateur est connectée électriquement à une première borne du premier dispositif de commutation, une électrode positive et une électrode négative de la première diode sont connectées électriquement à une deuxième borne du premier condensateur et à une deuxième borne du premier dispositif de commutation respectivement, et une borne de commande du premier dispositif de commutation est connectée électriquement à l'unité de commande (200).

8. Dispositif de surveillance d'impulsions (10) selon la revendication 7, dans lequel le premier circuit générateur d'impulsions (110) comprend en outre une deuxième diode ; une première borne d'un premier condensateur dans un premier niveau de première unité de génération d'impulsions (111) dans l'au moins un niveau de premières unités de génération d'impulsions (111) est connectée électriquement à une première source d'alimentation par le biais de l'au moins une deuxième diode.

9. Dispositif de surveillance d'impulsions (10) selon la revendication 7 ou 8, dans lequel le premier circuit générateur d'impulsions (110) comprend en outre au moins une troisième diode, une anode et une cathode de la troisième diode sont connectées électriquement à deux premières unités de génération d'impulsions (111) adjacentes respectivement.

10. Dispositif de surveillance d'impulsions (10) selon l'une quelconque des revendications 7 à 9, dans lequel le deuxième circuit générateur d'impulsions (120) comprend au moins un niveau de deuxièmes unités de génération d'impulsions (121) connectées électriquement de manière séquentielle ;

chaque deuxième unité de génération d'impulsions (121) étant connectée électriquement à l'unité de commande (200) et configurée pour être mise en marche sous la commande de l'unité de commande (200) et pour appliquer la deuxième séquence d'impulsions au tissu biologique cible.

11. Dispositif de surveillance d'impulsions (10) selon la revendication 10, dans lequel la deuxième unité de génération d'impulsions (121) comprend un deuxième condensateur, un deuxième dispositif de commutation et une quatrième diode ; une première borne du deuxième condensateur est connectée électriquement à une première borne du deuxième dispositif de commutation, une électrode positive et une électrode négative de la quatrième diode sont connectées électriquement à une deuxième borne du deuxième condensateur et à une deuxième borne du deuxième dispositif de commutation respectivement, et une borne de commande du deuxième dispositif de commutation est connectée électriquement à l'unité de commande (200).

12. Dispositif de surveillance d'impulsions (10) selon l'une quelconque des revendications 7 à 10, dans lequel l'unité d'affichage (400) est connectée en communication à l'unité de commande (200), l'unité d'affichage (400) étant configurée pour afficher au moins l'une parmi une valeur d'impédance en temps réel, une courbe de valeur d'impédance en temps réel ou des informations d'indicateur biologique.

13. Dispositif de surveillance d'impulsions (10) selon l'une quelconque des revendications 7 à 12, dans lequel le dispositif de surveillance d'impulsions comprend en outre une unité d'alarme (500) connectée en communication à l'unité de commande (200), l'unité d'alarme (500) étant configurée pour émettre une invite d'alarme lorsque les informations d'indicateur biologique du tissu biologique cible sont supérieures à un seuil défini.

Fig. 1

Fig. 2

Fig. 3

applying a first pulse sequence and a second pulse sequence to target biological tissue, where a voltage of the second pulse sequence is smaller than a voltage of the first pulse sequence, and the first pulse sequence is used to ablate the target biological tissue ⎯ S401

obtaining a feedback signal after the second pulse sequence is applied to the target biological tissue ⎯ S402

determining, according to the feedback signal, whether a termination condition for the first pulse sequence is met ⎯ S403

stopping applying the first pulse sequence when it is determined that the termination condition for the first pulse sequence is met ⎯ S404

Fig. 4

Fig. 5

Fig. 6

**EP 4 397 261 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 109157280 A